# EUROPEAN PATENT APPLICATION

(11) **EP 4 454 547 A1**
(43) Date of publication of application: **30.10.2024**
(21) Application number: 23169703.8
(22) Date of filing: 25.04.2023
(51) Int. Cl.: A61B 5/022, A61B 5/0235, A61B 5/021

(54) **AIR FLOW CONTROL FOR HEMODYNAMIC MEASUREMENT CUFF**

(71) Applicant: Koninklijke Philips N.V., 5656 AG Eindhoven (NL)
(72) Inventor: HILGERS, Achim Rudolf, 5656 AG Eindhoven (NL); WIJSHOFF, Ralph Wilhelm Christianus Gemma Rosa, 5656 AG Eindhoven (NL); DAVIDOIU, Valentina-Geta, 5656 AG Eindhoven (NL); SCHMITT, Lars, 5656 AG Eindhoven (NL); MENA BENITO, Maria Estrella, 5656 AG Eindhoven (NL)
(74) Representative: Philips Intellectual Property & Standards

(57) **Abstract**

A passive self-adjusting flow regulator adapted to vary a level of flow modulation in dependence upon a pressure of air in a fluid supply path inflating a bladder of a hemodynamic parameter measurement cuff. The flow regulator includes a structure which has a physical configuration arranged to vary as a function of fluid pressure in the fluid supply path, and wherein the varying physical configuration changes a level of flow modulation. One set of embodiments employs a self-regulating vent valve structure. A further set of embodiments employs a self-regulating fluid resistance structure.

## Description

### FIELD OF THE INVENTION

The present invention relates to the field of hemodynamic measurement using an inflatable cuff, and in particular to air flow control in such a context.

### BACKGROUND OF THE INVENTION

Hemodynamic monitoring refers to the measurement and analysis of blood flow characteristics in a vascular system of a patient. It is particularly important in the context of monitoring of critically ill patients as changes in blood flow and blood pressure can have a significant impact on health status and are indicative of various other physiological conditions of the patient. Hemodynamic monitoring in the critical context typically employs use of invasive measurement methods because these provide the most accurate measurements. Invasive methods involve for example arterial catheterization or pulmonary artery catheterization.

It would be of value in the medical field to provide non-invasive hemodynamic monitoring methods capable of rivalling the accuracy and reliability of invasive methods.

The most common non-invasive method of monitoring blood pressure and other hemodynamic measurements is through the use of an inflatable cuff attached to the arm of the patient. Such systems are known as sphygmomanometers.

### SUMMARY OF THE INVENTION

In seeking to develop improvements in cuff-based hemodynamic monitoring, the inventors associated with the present application have identified that it may be beneficial in some contexts to vary a size of an inflatable cuff which is used depending upon the arm size of the patient. It would be beneficial to provide a control system which is able to handle cuffs of different sizes (i.e., different bladder volumes) without interfering with the inflation cycle characteristics or the measurement algorithm. To this end, the inventors have recognized that some variation to the air flow characteristics of the pressurized air supply to the cuff is beneficial when changing cuff size. One way of achieving this is to modify control of the air flow generator. Another way of achieving this is to provide a dynamically controllable air flow modulator. However, both of these solutions are relative complex in operation. The inventors have recognized that there may be benefit in at least some circumstances for an air flow regulation means which is simpler.

The invention is defined by the independent claims. The dependent claims define advantageous embodiments.

According to examples in accordance with an aspect of the invention, there is provided a flow regulator for use along a pressurized fluid supply path between a pressurized fluid source and an inflatable bladder of a hemodynamic measurement cuff, wherein the flow regulator comprises:
a flow modulator configurable to provide variable level of flow modulation,
wherein at least a portion of the flow modulator has an adjustable physical configuration, wherein adjustment of the physical configuration varies the level of flow modulation;
wherein the flow modulator includes a pressure coupling area arranged to be in pressure-coupled relationship with the pressurized fluid flowing along the fluid supply path, and
wherein said physical configuration of the at least portion of the flow modulator varies passively as a function of the pressure acting on the pressure coupling area.

In particular, the flow regulator may be for use with a hemodynamic parameter measurement apparatus, the apparatus comprising: a source of pressurized fluid, a measurement cuff comprising an inflatable bladder, and a fluid supply path for conveying pressurized fluid from the source of pressurized fluid to the bladder of the measurement cuff; and wherein the flow regulator is for disposal along the fluid supply path between the pressurized fluid source and the inflatable bladder of the measurement cuff.

Another aspect of the invention provides a hemodynamic parameter measurement apparatus, comprising: a source of pressurized fluid; a measurement cuff comprising an inflatable bladder; a fluid supply path for conveying pressurized fluid from the source of pressurized fluid to the bladder of the measurement cuff; and a flow regulator as outlined above, or in accordance with any embodiment presented in this disclosure.

It is thus proposed to provide a flow regulation component with a pressure-coupling arrangement that allows for passive flow modulation as a function of pressure of the supply fluid. In this context, flow means the quantity of fluid that passes a particular point per unit time, i.e., volume per unit time. For example, the units of fluid flow may be Litres/second. It is also noted that the terms flow rate and flow may be used synonymously in this disclosure.

By providing a component in which the fluid pressure modulates a physical configuration of the component, and wherein the level of pressure or flow modulation is mechanically coupled with that physical configuration, this provides a fully passive regulation mechanism. As will become clear from later descriptions, there are a variety of particular ways to implement the concept in practice. However, the general concept of a level of flow modulation varying passively as a function of fluid pressure is common to all embodiments.

The degree of flow modulation varies as a function of pressure and this is effected through a passive regulation mechanism.

The flow modulation has the effect of modulating or varying or modifying a flow of the fluid supplied to the cuff bladder. In the context of this disclosure, a level of flow modulation generally means a level of flow reduction or suppression imposed by the flow modulator. Flow means rate of fluid passage per unit time (volume/time). It may be understood synonymously with flow rate. Providing flow modulation at a point along a flow path means varying/modulating the flow rate of fluid along the flow path.

Providing a variable level of flow modulation means providing a variable level of flow modulation across a range spanning between a minimum flow modulation (e.g. zero) and a maximum flow modulation. In other words, providing a variable level of flow modulation means providing a plurality of different possible flow modulation levels or states across the range between zero flow modulation and a maximum flow modulation. The flow modulator may be operable to provide a plurality of discrete levels of flow modulation or may provide a continuously variable level of flow modulation.

In some embodiments, the physical configuration of the at least portion of the flow modulator varies as a function of the pressure acting on the pressure coupling area such that the level of flow modulation decreases as a function of pressure. In other words, greater pressure leads to less flow modulation.

This configuration may be beneficial for example in circumstances in which a volume size of the inflatable bladder of the cuff may vary in different instances of operation. For a smaller cuff volume, it is preferable that a pressure or flow rate of fluid into the cuff at a beginning of the inflation cycle is reduced compared to a larger cuff so as to avoid inflating the cuff too rapidly. In other words, in order to provide a consistent rate of inflation across cuffs of different sizes (in terms of resultant pressure applied by the cuff to the body part), it may be preferable to provide smaller cuffs with a lower flow rate or lower pressure at a start of the inflation cycle than larger cuffs since there is a smaller volume to inflate. Rather than modifying a pressure or flow output of the fluid source, the flow modulator applies a modulation or variation to the flow rate at a point part way along the flow path, thereby changing an overall pressure of the system and a flow rate of fluid entering the cuff.

Toward the end of the inflation cycle, approximately the same pressure of air is needed for small cuffs compared with larger cuffs. Thus, there is advantage in a configuration in which the degree of flow modulation (and consequently the degree of pressure reduction) imposed by the flow regulator reduces in relationship with the pressure of the fluid in the fluid supply path to the cuff bladder. Thus, toward the end of the inflation cycle, when pressure to the cuff (and the overall system, including the fluid supply path) is highest, the degree of flow modulation is lowest.

In some embodiments, the adjustable physical configuration is an adjustable position of at least a part of said at least portion of the flow modulator. In other embodiments, the adjustable physical configuration is an adjustable dimension of at least a part of said at least portion of the flow modulator.

One main group of embodiments relates to use of a variable fluid release vent to modulate the fluid flow/pressure. As will be explained, this can be achieved in some examples with a single outflow orifice or a plurality of orifices, or with a semi-permeable wall section along the fluid supply path.

Thus, according to one or more embodiments, the flow modulator comprises a fluid release vent for venting fluid out from the pressurized fluid supply path, and wherein the outflow level from the vent may be variable to provide the variable level of flow modulation.

In some embodiments, the fluid release vent may comprise a closure structure, the closure structure having the previously mentioned adjustable physical configuration, and wherein adjustment of the physical configuration of the closure structure varies an outflow level from the vent. The closure structure may include the previously mentioned pressure coupling area.

Thus, in this set of embodiments, the flow regulator provides a self-regulating fluid release vent. The degree of closure of the vent varies passively as a function of pressure of the fluid in the fluid supply path.

In some embodiments, the physical configuration of the closure structure varies as a function of the pressure acting on the pressure coupling area such that the outflow level from the vent decreases as a function of pressure. This achieves a regime in which the flow level is reduced only when the pressure in the tube is lower. As discussed above, in practice, this has the effect that during the beginning part of the inflation cycle, when pressure is lowest, the flow is actively reduced by the vent. Toward later stages of the inflation cycle, when pressure is higher, flow is modulated to a lesser extent or not at all.

To achieve this, in some embodiments, there may be a biasing means which acts to bias the vent valve toward an open position in the absence of counterforce (i.e., relaxed state has the vent open), and wherein the closure structure acts to resist this bias with increasing resistance force as a function of increasing fluid pressure coupled from the fluid in the fluid supply path.

In some embodiments, the fluid release vent comprises an orifice, or an arrangement of multiple orifices, for outflow of fluid. The closure structure may comprise a seat member for seating over the orifice to seal or release the orifice to a variable extent. The adjustable physical configuration of the closure structure may comprise an adjustable positioning of the seat member relative to the orifice or arrangement of orifices, and wherein the seat member is variable across a range of positions for thereby varying an outflow level from the vent. Instead of an orifice, a permeable or semi-permeable wall section may be provided. The orifice is for example formed in a wall of a conduit comprised by the flow regulator which is connected or integrally disposed in series along the fluid supply path.

In some embodiments, the pressure coupling area may be an area of a moveable member, the moveable member arranged to move by a variable amount related to an applied pressure from the fluid, and wherein the movement of the moveable member is coupled directly or indirectly to the seat member. Optionally, the closure structure may comprise a biasing element (e.g., a spring element) for biasing the seat member into an open position relative to the orifice in the absence of counter-force, and wherein the movement of the moveable member responsive to applied pressure acts to counter the biasing element to move the seat member into a more closed position. In other words, greater fluid pressure leads to reduced venting.

In some embodiments, the moveable member may comprise a flexible membrane.

In some embodiments, the moveable member may comprise a linearly moveable plate. This may provide a piston arrangement.

In some embodiments, the moveable member itself forms or comprises the seat member, e.g. by providing a flexible membrane which itself seats against an orifice.

In some embodiments, the moveable member is a pivotable flap with a seat member mounted or carried at a moveable end of the flap.

In some embodiments, the moveable member is arranged in pressure-coupled arrangement on one side with the pressurized fluid flowing along the fluid supply path and is in pressure-coupled arrangement on an opposite side with a reference pressure, e.g. an ambient air pressure, whereby a pressure differential is established across said two sides of the moveable member. For example, a flexible membrane may be provided in fluid communication with the fluid supply tube on one side and with a reference pressure chamber on the other side.

In some embodiments, the function of the change in the physical configuration of the closure structure with pressure acting on the pressure coupling area is adjustable. For example, where the closure structure comprises a biasing element (e.g. a spring) the biasing strength may be adjusted.

In some embodiments, a non-linear force transfer means is provided between the pressure coupling area and the closure structure. This has the effect of providing a non-linear relationship between fluid pressure in the fluid supply path and the degree of flow modulation by the flow modulator. For example, where the closure structure comprises a biasing element for biasing a seat member into an open position relative to the orifice in the absence of counter-force, this biasing element may be provided as a non-linear biasing element.

In one set of embodiments, the flow modulator includes a tubing piece for location along the fluid supply path, and wherein at least a section of the tubing piece is flexibly deformable as a function of fluid pressure inside the section of the tubing piece. The at least section of the tubing piece may include one or more fluid outflow paths, to permit venting of fluid out from the fluid supply path, for example one or more orifices or conduits formed in a wall of the tubing, or a permeable wall area. The pressure coupling area may be formed by an interior, fluid-facing surface of the section of the tubing piece. The flow modulator may further include a seat member mounted outside of the tubing piece and facing the one or more outflow paths. The tubing piece may be flexibly deformable between a relaxed state in which there is a spacing between the seat member and the one or more outflow paths, and a deformed state in which the one or more outflow paths are closed against the seat member.

Thus here, the section of the tubing piece has the adjustable physical configuration by virtue of being deformable, and an outflow from e.g. one or more orifices is passively regulated via expansion of the tubing toward the seat member outside the tubing.

In some embodiments, the aforementioned seat member may comprise a sleeve member having lesser flexibility than the section of the tubing piece, e.g. a rigid or inflexible sleeve member.

In a further set of embodiments, the flow modulator includes a tubing piece for location along the fluid supply path, and wherein at least a section of the tubing piece is flexibly deformable as a function of fluid pressure inside the section of the tubing piece; and wherein the at least section of the tubing piece has a permeable or semi-permeable wall. The deformation of the tubing responsive to pressure may vary a permeability of the wall. In other words, the deformation of the wall changes a size of orifices or conduits formed through the wall, thereby changing an outflow level from the tubing.

Various embodiments outlined above relate principally to a configuration in which the flow modulation is achieved by means of passively self-adjusting venting of gas. A further group of embodiments is based on a configuration comprising a fluid resistor.

In particular, in some embodiments, the flow modulator comprises a fluid resistor for imposing a resistance against a flow of the pressurized fluid through the fluid supply path. A level of resistance of the fluid resistor may be variable to provide the variable level of flow modulation. The fluid resistor may include a flow interruption structure for impinging on fluid flowing along the fluid supply path, the flow interruption structure having said adjustable physical configuration, and wherein adjustment of the physical configuration of the flow interruption structure varies a level of fluid resistance imposed by the fluid resistor. The flow interruption structure may include the previously discussed pressure coupling area.

In some embodiments, the fluid interruption structure may comprise one or more fluid flow paths extending from one side of the interruption structure to another to permit fluid to pass therethrough, and wherein the adjustment of the physical configuration of the interruption structure includes adjustment of a cross-sectional area of said one or more fluid flow paths.

For example, in some embodiments, the flow interruption structure may comprise a flexibly deformable body, the fluid flow paths being formed through the deformable body, and wherein the fluid flow paths are configured to flexibly deform as a function of fluid pressure inside the section of the tubing piece. This provides an efficient mechanism for passive flow regulation. Higher pressure automatically leads to expansion of the flow paths, and reduced flow resistance. Thus, a level of flow modulation (resistance) decreases with increasing flow pressure.

In some embodiments, the flexibly deformable body of the flow interruption structure is mechanically coupled to an interior wall of the at least section of the tubing piece, such that deformation of the tubing wall effects a correlated deformation of the interruption flow structure. By way of one example, the flexibly deformable structure may be a flexible membrane which is attached at its periphery to an interior surface of the tubing piece.

In some embodiments, the fluid interruption structure may be configured to extend across a whole of a cross-sectional area of the fluid supply path. Thus, the width and number of the fluid flow paths may determine the fluid resistance.

Another aspect of the invention is a hemodynamic parameter measurement apparatus, comprising: a source of pressurized fluid; an inflatable hemodynamic measurement cuff; a fluid supply path for conveying pressurized fluid from the source of pressurized fluid to the inflatable cuff; and a flow regulator in accordance with any embodiments described in this document disposed along the fluid supply path for modulating flow of the pressurized fluid.

In some embodiments, the apparatus may comprise a first and second cuff, the second cuff having a smaller bladder (e.g. smaller bladder volume) than the first; and wherein the system is selectively operable using either the first or second cuff connected to fluid supply path. For example, the second cuff may be designed for application to a child and is designed for wrapping around a body part with smaller outer diameter.

In some embodiments, the measurement cuff of the apparatus comprises a tissue pressure sensor arranged for sensing a pressure between a surface of the body part and the bladder of the cuff.

In some embodiments, the cuff includes a shell portion, wherein the shell portion is arranged so as to be located between the inflatable bladder and the body part when the cuff is wrapped around the body part; wherein the shell portion is arranged to surround the body part when the cuff is wrapped around the body part.

In some embodiments, the hemodynamic parameter measurement apparatus further includes a measurement module for processing a pressure signal output by the cuff and generating one or more blood pressure measurements.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of an example apparatus in accordance with one or more embodiments of the invention;
Fig. 2 schematically illustrates a flow regulator in accordance with one set of embodiments in which a vent valve principle is employed;
Fig. 3 schematically illustrates a flow regulator in accordance with a further set of embodiments in which a vent valve principle is employed;
Fig. 4 schematically illustrates a flow regulator in accordance with a further set of embodiments in which a vent valve principle is employed;
Fig. 5 schematically illustrates a flow regulator in accordance with a further set of embodiments in which a vent valve principle is employed;
Figs. 6-7 schematically illustrates example flow regulators in accordance with a further set of embodiments in which a semi-permeable tubing is employed;
Fig. 8 schematically illustrates a further example flow regulator in accordance with one or more embodiments;
Fig. 9 schematically illustrates a further example flow regulator in accordance with one or more embodiments;
Fig. 10 schematically illustrates a flow regulator in accordance with a further set of embodiments in which a variable flow resistance principle is employed; and
Fig. 11 schematically illustrates a flow regulator in accordance with a further set of embodiments in which a variable flow resistance principle is employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a passive self-adjusting flow regulator which varies a level of flow modulation in dependence upon a pressure of air in a fluid supply path/system inflating a bladder of a hemodynamic parameter measurement cuff. The flow regulator includes a structure which has a physical configuration arranged to vary as a function of fluid pressure in the fluid supply path, and wherein the varying physical configuration changes a level of flow modulation. One set of embodiments employs a self-regulating vent valve structure. A further set of embodiments employs a self-regulating fluid resistance structure.

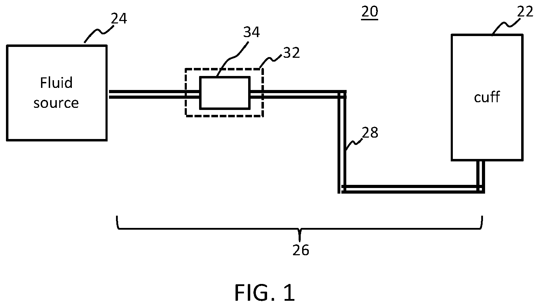

Fig. 1 illustrates components of an example apparatus according to one or more embodiments. The apparatus 20 comprises a hemodynamic measurement cuff 22 with an inflatable bladder for adjusting a pressure applied by the cuff to a body part of a patient around which the cuff is worn.

The apparatus 20 further comprises a source 24 of pressurized fluid (e.g. air or another gas) for inflating the bladder of the cuff. For example, this may comprise a pressurized air generator. For example, this may comprise an air pump or an air condenser.

Connecting the fluid source 24 and the bladder of the cuff 22 is an air flow path or fluid supply path 26 for conveying pressurized fluid from the source of pressurized fluid 24 to the inflatable cuff 22. The fluid supply path may be facilitated by a plurality of different components and sections, which may include one or more conduits, tubes, hoses, chambers, reservoirs, valves and so on. For example, some portions of the fluid supply path may be integrated in a housing which houses the fluid source, and some may be formed by the tubing 28 which connects to the cuff 22.

Disposed in-line along the fluid supply path, e.g. in fluidic series, is a flow regulator 32.

The flow regulator 32 comprises a flow modulator 34 which is configurable to provide a variable level of flow modulation. Flow modulation means to modify the flow of fluid passing along the fluid supply path to the bladder of the cuff. It may involve decreasing the rate of flow so that a variable level of flow modulation amounts to a variable level of flow reduction or suppression. It may have the effect of adjusting a pressure of the fluid flowing through the fluid supply path to the bladder of the cuff.

The flow modulator 34 is configurable to provide a variable level of flow modulation across a range of flow modulation states spanning between a minimum flow modulation and a maximum flow modulation. In other words, the flow modulator is configurable in a plurality of flow modulation states within the range between minimum (e.g. zero) and maximum flow modulation.

The flow modulator may be operable to provide a plurality of discrete levels of flow modulation or may provide a continuously variable level of flow modulation.

The flow regulator 32 may be removably connectable into the fluid supply path or may be fixedly integrated in the fluid supply path. The flow regulator is arranged so that fluid flows through the flow modulator 34 en route to the bladder of the cuff. The flow regulator may include inflow and outflow connectors for connecting to corresponding connectors of the fluid supply path 26 such that fluid flows through the flow modulator 34 en route to the bladder of the cuff.

At least a portion of the flow modulator 34 has an adjustable physical configuration, wherein adjustment of the physical configuration varies the level of flow modulation provided to the fluid flowing along the fluid supply path.

The flow modulator 34 includes a pressure coupling area arranged to be in pressure-coupled relationship with the pressurized fluid flowing along the fluid supply path.

The physical configuration of the at least portion of the flow modulator 34 varies passively as a function of the pressure acting on the pressure coupling area.

The flow regulator 32 can be provided by itself as one aspect of the invention.

Another aspect of the invention is an apparatus 20 comprising the flow regulator 32 in combination with one or more of the further components of the example apparatus of Fig. 1, e.g. the pressurized air source 24, the fluid supply path 26 and/or the cuff 22 with inflatable bladder.

In some embodiments, the apparatus may comprise a plurality of cuffs, each having an inflatable bladder of a different inflatable volume capacity, and wherein the apparatus 20 is selectively operable using any one of the cuffs. In other words, each cuff is fluidly connectable to the fluid supply path from the pressurized fluid source.

For example, the apparatus may include a first and second cuff, the second cuff having a smaller bladder than the first, and wherein the apparatus is selectively operable using either the first or second cuff connected to the air flow path.

The structure and operation of the flow regulator 32 will become clearer with reference to example embodiments, description of which will follow.

At least two broad groups of embodiments might be identified: a first group in which the flow modulator operates on a principle of venting fluid in order to modify flow (i.e. automatically releasing superfluous air), and a second group which operates on a principle of restricting flow through a flow resistance element.

Various example embodiments in accordance with the first general group will first be outlined.

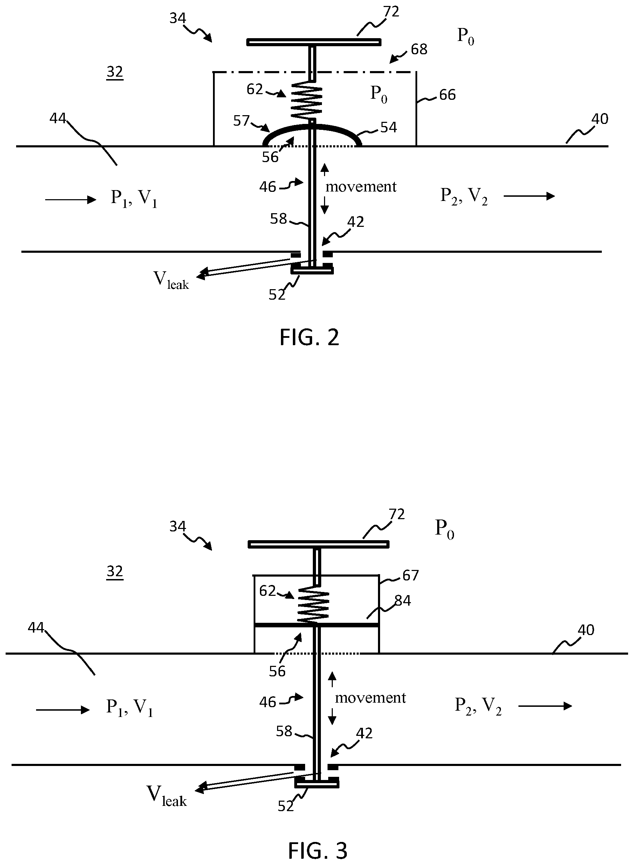

Reference is made to Fig. 2 which schematically illustrates the structure and operation of a first example flow regulator 32 in accordance with one or more embodiments. The flow regulator includes a flow modulator, generally indicated by arrow 34. The flow modulator in this example comprises a fluid release vent for venting fluid out from the pressurized fluid supply path 44, wherein an outflow level from the vent is variable to provide a variable level of flow modulation by the flow modulator 34 as a whole.

To facilitate controlled variation in the outflow level from the vent, the flow modulator 34 comprises a closure structure 46, the closure structure having an adjustable physical configuration, wherein adjustment of the physical configuration of the closure structure varies an outflow level from the vent. The fluid release vent in this example comprises an orifice 42 for outflow of fluid from the pressurized fluid supply path 44 and the closure structure 46 comprises a seat member 52 being seatable over the orifice 42 to seal or release the orifice to a variable extent.

The physical configuration of the closure structure 46 varies as a function of pressure within the pressurized fluid supply path 44. More particularly, the adjustable physical configuration takes the form of an adjustable positioning of the seat member 52 relative to the orifice 42, and wherein the seat member 52 is variable across a range of positions for thereby varying an outflow level from the vent.

To facilitate coupling between the pressure in the fluid supply path 44 and the positioning of the seat member 52 relative to the orifice 42, a moveable member is provided in the form of a flexible membrane 54. An inward-facing surface 56 of the flexible membrane 54 (meaning a surface facing toward the fluid supply path 44) forms a pressure coupling area which is pressure-coupled with the pressurized fluid supply path 44. This means that variations in pressure of fluid within the fluid supply path 44 effect changes in position of the flexible membrane 54. The flexible membrane 54 is mechanically coupled with the seat member 52 by means of a mechanical coupling element 58. For example, in the illustrated example of Fig. 2, the mechanical coupling element is a shaft member 58. In the illustrated example, the flexible membrane is pressure coupled with the fluid supply path via a fluid-permeable area in a side wall of a conduit 40 which defines the fluid supply path.

In particular, the flexible membrane 54 is in pressure-coupled arrangement on one side 56 with the pressurized fluid flowing along the fluid supply path 44 and is in pressure-coupled arrangement on an opposite side with a reference pressure whereby a pressure differential is established across said two sides of the moveable member. In the illustrated example, the reference pressure is an ambient air pressure, Po. This is achieved by providing a reference pressure chamber 66, and wherein the flexible membrane is in fluid communication on one side with the fluid in the fluid supply path 44 and in fluid communication on the other side with fluid (air in this example) in the reference pressure chamber. In effect, the membrane 54 fluidly divides the reference pressure chamber 66 from the fluid supply path 44 (in the conduit 40). In this example, the reference pressure chamber is fluidically open to the ambient atmosphere, e.g. via a fluid permeable wall 68, so that the reference pressure in the chamber 66 is ambient/atmospheric air pressure, Po.

The pressure differential between the reference pressure chamber 66 and the fluid supply path 44 determines the positioning of the membrane 54 (i.e. the moveable member) and thus the positioning of the seat member 52 relative to the orifice 42, and thus the outflow level from the vent.

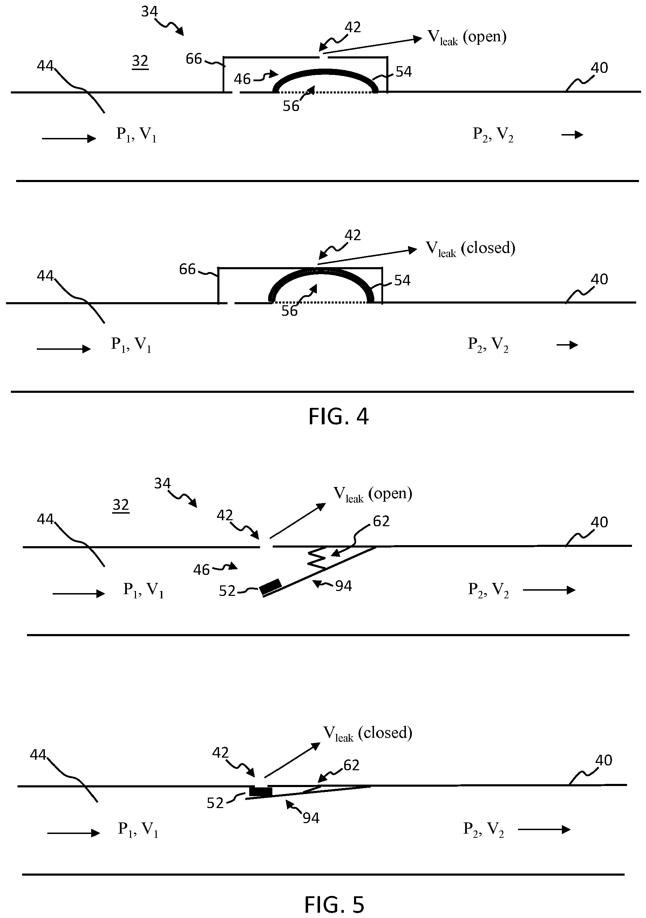

In the illustrated example of Fig. 2, the flexible membrane 54 is disposed on an opposite side of the conduit 40 to the orifice 42 and thus the mechanical coupling element 58 is provided to facilitate mechanical coupling between the two. As will become clear when describing later embodiments, e.g. with reference to Fig. 4 and Fig. 5, this arrangement is not essential.

In the illustrated example, the closure structure 46 comprises a biasing element 62, e.g. in the form of a spring element, for urging against the membrane 54 in such a way as to bias the seat member 52 (mechanically coupled to the membrane) into an open position relative to the orifice 42 in the absence of counter-force. The movement of the membrane 54 responsive to applied pressure acts to counter the biasing element 62 to move the seat member into a more closed position. In other words, the biasing elements acts to pre-condition (load) the membrane.

In the illustrated example, the shaft 58 mechanically coupling the membrane 54 to the seat member 52 comprises the seat member at one end of the shaft and a stop member 72 an opposite end, the opposite end protruding through an opening formed through the upper wall 68 of the reference pressure chamber 66, and the stop member acting to define a maximum movement of the seat member 52 away from the orifice 42.

It is noted that another possible function of the stop member 72 may be to adjust the stiffness of the spring. Different spring stiffnesses provides freedom to (pre-)define the air release rate as a function of pressure.

Advantageously, the reference pressure chamber 66 is also utilized to house or contain the biasing element 62, wherein a wall 68 of the reference pressure chamber acts as a grounding or support surface against which the biasing element can be compressed by the membrane 54.

The described arrangement has the effect that the orifice is more open during a low pressure phase and more closed during a high pressure phase. Thus, for example, during a time period at a beginning of a cuff inflation cycle, where pressure is relatively low, greater venting is provided, while during a period toward an end of a cuff inflation cycle, where pressure is relatively higher, lesser venting is provided.

To illustrate the action of the flow regulator 32, an example of operation of the structure will now be described.

As illustrated in Fig. 2, the fluid in the air flow path 44 can be modelled as having fluid pressure P = P₁ = P₂, where P₁ is the pressure on an upstream side of the flow modulator 34 and P₂ is the pressure on a downstream side of the flow modulator 34. A flow rate (m³/s) of fluid upstream from the flow regulator (arriving from the pressurized air source), Vi, can be modelled as being greater than the flow rate of the fluid downstream from the flow regulator, V₂, by an amount equal to Vₗₑₐₖ, which represents a flow rate of fluid leaking out through the orifice 42. Thus, once equilibrium is established, the pressure of fluid on both sides of the air regulator, for example across the whole fluid system, can be understood to be approximately uniform, but wherein a flow rate of fluid may differ upstream and downstream of the flow regulator, wherein the flow regulator changes the flow, and wherein the level of flow modification affects said overall pressure in the fluid system.

In the illustrated example, the fluid is gas, preferably air.

The pressure differential, ΔP, across the two sides of the membrane 54 can in this example be represented as ΔP = P- Po - Pₜₕ, where Pₜₕ is a threshold pressure, defined by the load exerted by the biasing element 62 and Po is the reference pressure in the reference pressure chamber 66, i.e. ambient pressure in this particular example.

At the start of an inflation cycle for inflating the cuff (to = 0) the air-outlet (orifice 42) is open because the biasing element 62 pushes the membrane 54 downwards, thereby holding the seat member in an open position relative to the orifice 42. While inflating the cuff 22, air enters the cuff (at flow rate V₂) but is also released via the open orifice (at flow rate Vₗₑₐₖ). As a result, the pressure (P) in the cuff slowly increases. As long as ΔP is smaller than the sum of the pre-defined threshold pressure Pₜₕ (defined by the rigidity of the membrane and the pre-condition or biasing mechanism 62) and the reference air pressure (Po), i.e., ΔP < 0 or Po + Pₜₕ ≫ P, air will be further released via the orifice 42.

With increased pressure (P) in the conduit 40 and cuff 22 system, the pressure difference ΔP becomes smaller, i.e., less negative, and accordingly the position of the membrane 54 (defined e.g. by an expansion of the membrane due to ΔP) changes to push further against the biasing element 62 and consequently move the seat member 52 into a progressively more closed position relative to the orifice 42. Accordingly, the flow rate of leaked air flowing out the orifice 42 (Vₗₑₐₖ) decreases and finally becomes zero if ΔP reaches or rises above the sum of the threshold pressure (Pₜₕ) and the reference air pressure (Po), i.e., ΔP ≥ 0 or Po + Pₜₕ ≪ P. Thereafter, the air flow rate into the cuff (V₂) is equal to the air flow entering the tube (Vi), causing the cuff to be pressurized more quickly (assuming a constant air flow Vi).

The thus described automatic air release means has the additional advantage, that during the deflation of the air cuff 22, the deflation cycle can be shortened since additional air can be released via the orifice, in particular at low cuff pressures (P < Pₜₕ + P₀).

In the example of Fig. 2, the moveable member is provided as a flexible membrane 54 which urges against the biasing element to effect variation in a position of the seat member 52 relative to the orifice 42. However, alternative arrangements are possible.

Fig. 3 shows a further example in which the moveable member comprises a linearly moveable plate 84. The principle of operation may otherwise be similar to the example of Fig. 2.

In particular, there is provided a closure structure 46 with a configurable physical configuration and wherein the closure structure comprises a seat member 52 for seating over the orifice 42 to seal or release the orifice to a variable extent. The adjustable physical configuration of the closure structure comprises an adjustable positioning of the seat member 52 relative to the orifice 42, wherein the seat member is variable across a range of positions for thereby varying an outflow level from the vent.

The moveable member in the form of the plate 84 has inner-facing surface 56 which acts as the pressure coupling area, and wherein the moveable member is arranged to move by a variable amount related to an applied pressure from the fluid in the fluid flow path 44. The movement of the moveable member 84 is mechanically coupled to the seat member 52. The closure structure 46 also includes a biasing element 62, such as a spring, for biasing the seat member 52 into an open position relative to the orifice in the absence of counter-force. The movement of the moveable plate member 84 responsive to applied pressure acts to counter the biasing element to move the seat member into a more closed position.

The plate member 84 effectively forms a piston within a surrounding cylinder 67. The piston is moveable up or down within the cylinder and this movement varies a positioning of the seat member 52 relative to the orifice 42. The movement of the piston controls the level of outflow from the orifice 42 and thus controls the level of flow modulation applied by the flow modulator 34 as a whole. Pre-conditioning of the plate 84 is achieved by the biasing element 62, e.g. a spring, in combination with a reference air-pressure in the part of the cylinder 67 which lies on a side of the plate 84 facing away from the fluid flow path 44. The plate, similar to the membrane in the example of Fig. 2, is exposed on one side 56 to the pressurized fluid in the fluid supply path 44, and exposed on the opposite side to a reference pressure, this time defined within the cylinder 67. Thus the cylinder 67 can also be understood as an example of a reference pressure chamber, similar to that used in the example of Fig. 2. One option is to make the reference pressure in the cylinder 67 equal to ambient air pressure Po, e.g. by making the cylinder fluidically open to the atmosphere, similar to the configuration of the example of Fig. 2. In this case, the pre-conditioning is defined only by the biasing element (and the ambient atmosphere).

However, it is also possible to provide a reference pressure in the cylinder 67 which is different from ambient air pressure. Indeed, this option is also applicable for the reference pressure chamber 66 of the example Fig. 2, wherein instead of ambient pressure, the chamber can be closed, and a defined reference pressure provided inside the chamber.

This principle is schematically illustrated in Fig. 3, which shows the cylinder 67 being closed to the ambient atmosphere.

The embodiment of Fig. 3 can instead be modified so that the cylinder 67 is open to the atmosphere such that the reference pressure in the cylinder is equal to ambient, Po.

To explain further, in a configuration where the pressure chamber 67 or cylinder 66 is open to ambient air pressure, then the biasing exerted on the moveable member 54, 84 has a clear dependency on the ambient air pressure (Po). This has the consequence that a change of the ambient air pressure would have an impact on the relationship between the fluid pressure in the fluid supply path 44 and the degree of flow modulation provided by the flow modulator 34. Therefore, to improve the accuracy of the automatic air release means, the air pressure may be pre-defined or measured.

In other words, the dependency of the flow regulator 32 on the ambient air pressure can be reduced if the space surrounding the biasing element 62 and the moveable member 54, 84 is a closed and sealed space/volume with a pre-defined reference pressure (P_{ref}). This could either be a fixed pressure defined during the production process or could be a variable pressure pre-defined by the operator or even automatically adjusted by the patient monitor or any other device connected to the flow regulator 32. In the latter case, the biasing element could even be omitted altogether.

Thus, as a more general principle, in accordance with one or more embodiments the moveable member 54, 84 may be provided in pressure-coupled relationship on one side with the pressurized fluid flowing along the fluid supply path 44 and in pressure-coupled arrangement on an opposite side with a reference pressure, e.g. an ambient air pressure, whereby a pressure differential is established across said two sides of the moveable member.

In some embodiments, the reference pressure in the pressure chamber 66 or cylinder 67 may be actively adjusted so as to vary the function of flow modulation with pressure. This in turn would lead to a modification in the inflation slope as a function of time of the cuff, assuming that the control of the air supply from the fluid source is left unchanged. Therefore, by means of controlling/adapting the reference pressure, the inflation ramp may be customized, e.g., can be made patient or disease (or cuff) specific. Thus, in some embodiments, the reference pressure in the pressure chamber 66 or cylinder 67 may be dynamically adjustable.

Thus, in some embodiments, the function of the change in the physical configuration of the closure structure with pressure acting on the pressure coupling area is adjustable. This can be achieved by changing the reference pressure in the pressure chamber or by e.g. by changing a biasing of the biasing element 62.

In some embodiments, the biasing element may be provided as a non-linear biasing element, e.g. a non-linear spring (e.g., a conical or tapered spring). By using a non-linear biasing element, the function between fluid pressure and level of venting can be controlled. In particular, while a linear spring has a linear relation between force and displacement, a nonlinear spring can be designed to provide any arbitrary nonlinear force-displacement relation, with resulting modifications to the inflation ramp.

From this can further be proposed the more general concept that, in some embodiments, a non-linear force transfer means is provided between the pressure coupling area 56 and the remainder of the closure structure 46.

Fig. 4 illustrates a further example in accordance with one or more embodiments.

In accordance with this embodiment, a flexible membrane 54 is provided, similar to the embodiment of Fig. 2, but wherein the orifice 42 is relocated relative to the membrane such that the membrane itself forms the seat member for seating over the orifice 42 to control an outflow level from the orifice 42.

In other words, the moveable member 54 itself directly forms the seat member of the vent valve.

In the illustrated example of Fig. 4, the moveable member is a flexible membrane 54 and wherein expansion of the membrane directly alters a level of outflow from the orifice 42. As pressure in the flow path 44 increases, a bending radius of the membrane increases, thereby progressively reducing an outflow level from the orifice by progressively closing the orifice.

However, as an alternative to the membrane 54, also the moveable plate member 84 of the example of Fig. 3 could be provided as the moveable member in this embodiment, wherein the plate member directly seats against the orifice to a variable extent to vary a level of outflow from the orifice 42.

In the arrangement of Fig. 4, the orifice is provided on a same side of the conduit 40 as the moveable member 54.

Fig. 5 shows a further example flow regulator 32 in accordance with one or more embodiments. In this example, the closure structure 46 takes the form of a spring-loaded flap 94. In this set of embodiments, the moveable member takes the form of a flap, and wherein the flap carries a seat member 52 for seating against the orifice 42 for varying a level of outflow from the orifice. The seat member 52 is disposed at a moveable end of the flap 94. In this example, the pressure coupling area is provided by a surface of the flap member which faces into the fluid flow path 44 defined by the conduit 40. Dependent on a value of the pressure of the pressurized fluid in the fluid supply path 44, the flap 94 will pivot up or down (toward or away from the orifice 42 respectively) by a correlated amount. In other words, the flap is arranged to move by a variable amount related to an applied pressure from the fluid, and wherein the movement of the flap is coupled directly to movement of the seat member 52 by virtue of the seat member being mounted at a distal end of the flap. The flap is joined by a pivotal joint or coupling to a wall of the conduit 40, permitting pivotal swinging of a distal end of the flap. The closure structure 46 further comprises a biasing element 62 for biasing the flap 94 (and thus also the seat member 52) into an open position relative to the orifice 42 in the absence of counter-force, and wherein the movement of the flap 94 responsive to applied pressure from the fluid 44 acts to counter the biasing element to move the seat member 52 into a more closed position.

Thus, in other words, in this set of embodiments, the passive control of the orifice is realized by means of a (sealing) flap 94. Fig. 5 illustrates the working principle, wherein the orifice 42 is variably sealable by the flap 94 situated at a point along the fluid supply path 26 between the pressurized fluid source 24 and the cuff 22. At a low fluid pressure in the fluid path 44, the flap 94 is pushed by means of the biasing element 62 (e.g. spring) away from the orifice 42. With increased pressure in the conduit, the flap 94 is progressively pushed towards the orifice 42 until finally closing the orifice once the fluid pressure reaches a threshold level. Although the example of Fig. 5 shows the distal end of the flap pointed in a direction opposite to the direction of flow of the fluid 44, the alternate arrangement would also be possible, wherein the distal end of the flap points in the same direction as the fluid flow. Indeed, this may even be the preferred solution since it may optimize the coupling of fluid pressure to the flap.

According to a further set of embodiments, the flow modulation functionality may be provided by an integral structural feature of a conduit or tubing carrying the pressurized fluid 44.

For example, according to one set of examples, the flow modulator may include a conduit or tubing piece for location fluidly in-line along the fluid supply path 26 between the fluid source 24 and the cuff 22, and wherein at least a section of the tubing piece is flexibly deformable as a function of fluid pressure inside the section of the tubing piece. The at least section of the tubing piece may further be provided having a permeable or semi-permeable wall, wherein the deformation of the tubing responsive to pressure varies a permeability of the wall. For example, a permeable or semi-permeable wall may refer to a wall which incorporates a plurality of flow paths, e.g. holes or openings or orifices, wherein optionally these are microsized. The permeability of the wall effects the level of flow modulation through escape of fluid through the semi-permeable wall area. Variation in a level of flow modulation is achieved by variation in an expansion state of the tubing piece wall, wherein this variation in the expansion state changes a size of the openings forming the permeability of the wall, to thereby change an outflow level from the permeable or semi-permeable wall area. Thus, the adjustment in the physical configuration in this set of examples corresponds to adjustment in an expansion state of the tubing piece. The pressure coupling area of the flow modulator in this set of examples is an internal surface of the tubing piece.

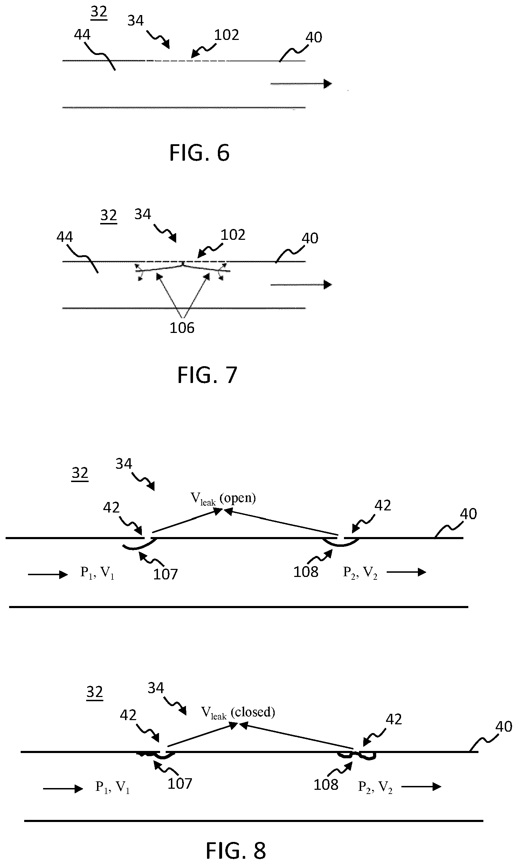

An example is illustrated schematically in Fig. 6. This illustrates an example conduit or tubing section 40, wherein a wall of the tubing section comprises a fluid permeable or semi-permeable area 102. For example, the permeable area may span a sub-portion of the length of the tubing section and a sub-portion of a circumferential extension of the tubing section. Air is able to escape in a controlled way from the semi-permeable wall portion 102. In this configuration, the majority of inflowing air will flow through the tubing section 40 without escaping. However, a portion of the air will be vented out of the tubing, thereby modifying an air flow rate along the tubing after the permeable wall section.

The variability in the wall permeability can be realized by providing the tubing section formed of a flexible material to permit variation in a size of the openings forming the semi-permeable section.

The flow regulator 32 may comprise just a single semi-permeable wall portion 102 formed at a single location along the fluid supply path, or the flow regulator may comprise a plurality of the semi-permeable wall portions disposed at a sequence of different locations along the fluid supply path 44.

Additionally or alternatively, as illustrated in Fig. 7, the flow modulator 34 may further include one or more membrane elements 106 which are configured to be moveable through a range of positions spanning between a first position in which the one or more membrane elements are seated against the semi-permeable wall portion 102 in order to seal the wall portion to prevent escape of fluid, and a second position in which the one or one or more membrane elements are suspended away from the wall portion so that fluid can escape through the wall portion. The variation in position of the one or more membranes may be responsive to pressure so that, at low pressure, the membranes are not seated against the wall portion and at higher pressure, the membranes are seated against the wall portion. The level of outflow from the semi-permeable wall portion 102 is a function of position of the one or more membrane elements, which is in turn a function of pressure of fluid in the fluid supply path 44.

This approach is compatible with a tubing section which is deformable, as in the example of Fig. 6, or which is non-deformable (e.g. rigid). By way of example, this set of embodiments can be understood as providing a flow modulator which comprises a fluid release vent, and wherein the membrane elements 106 form a closure structure having an adjustable physical configuration, wherein adjustment of the physical configuration of the closure structure varies an outflow level from the vent. Instead of the fluid release vent being defined by a single orifice, as in previously described examples, the fluid release vent is formed by a semi-permeable area 102 of the wall of a tubing section 40.

When used in operation, during a phase of low pressure inside the tubing section, the flexible membrane elements 106 would be "open" in the sense that they would not cover the permeable material, allowing air to escape via the permeable material 102, thereby reducing the air flow rate towards the cuff. At higher pressure inside the tubing, the flexible membranes would be "closed" in the sense that they would cover the permeable material, thereby not modulating the flow at all and allowing maximum airflow towards the cuff.

A variation on this approach is illustrated schematically in Fig. 8. This shows a tubing section which may or may not be deformable. The tubing section may include a semi-permeable wall portion and/or may comprise a plurality of orifices or holes 42. The flow modulator 34 may further include one or more membrane elements 107, 108 which are configured to each be moveable through a range of positions spanning between a first position in which the membrane element is seated against the orifice in order to seal the orifice to prevent escape of fluid, and a second position in which the membrane element is suspended away from the orifice so that fluid can escape through the tubing section 40.

The variation in position of each membrane element 107, 108 is responsive to pressure so that, at low pressure, the membrane elements are not seated against the orifices and, at higher pressure, the membrane elements are seated against the orifices.

This embodiment may be understood as similar to the embodiment of Fig. 5, except that the flaps 94 are replaced by the membrane elements. This may offer a structurally simpler solution. Each membrane element may be formed of a strip of flexible (e.g. elastomeric) material. The stiffness of each element can be selected in dependence upon the desired relationship between pressure and flow modulation. A surface of the membrane element may form the pressure coupling area.

Fig. 8 shows an example of each of two possible configurations for the membrane element. A first membrane element 107 is shown having one end which is fixedly attached to the tubing 40 wall and wherein the other end is free. The stiffness of the material may bias the membrane element to the second position in which the membrane element is suspended away from the orifice 42. Upon increase in pressure the free end of the membrane element 107 is progressively pushed upward so that the membrane element progressively closes against the orifice. Thus, flow modulation is reduced in proportion with greater fluid pressure in the tubing.

A second example membrane element 108 is also shown having both ends fixedly attached to the tubing wall at respective points positioned either side of the orifice. The stiffness of the material may bias the membrane element 108 to a position in which it is suspended away from the orifice 42. As pressure in the tubing increases, the middle section of the membrane element is progressively pushed toward the orifice, thereby progressively closing the orifice.

Although only two membrane elements are shown in Fig. 8, in a practical embodiment, more than two may be provided. Furthermore, although in the example of Fig. 8, each membrane element is shown as closing against a respective orifice, alternatively, each membrane element may be arranged to close against a respective semi-permeable wall portion. Furthermore, in some embodiments, all of the membrane elements may be of the first type 107 (with a single attachment point), or all of the membrane elements may be of the second type 108 (with two attachment points), or the flow modulator may comprise a mix of the two types of membrane element.

Furthermore, in some embodiments, a plurality of membrane elements may be provided distributed along the length of a tubing section 40, each arranged for modulating flow through a respective orifice or permeable wall portion, and wherein each membrane element is provided having a different level of pressure-responsiveness so that each membrane element closes at a different rate as a function of fluid pressure. For example, this could be achieved by providing each membrane element with a different stiffness. This option allows for providing the overall flow modulator 34 with a more complex function of flow modulation level to fluid pressure, for example a non-linear function.

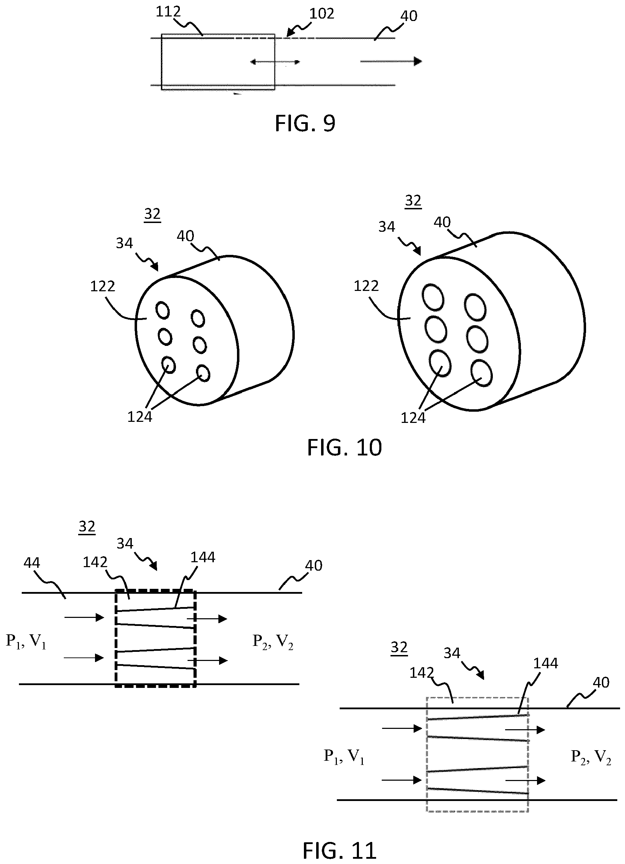

Fig. 9 schematically illustrates a further example in accordance with one or more embodiments. This example may take the same form as the embodiment of Fig. 6, except for the additional provision of a moveable member in the form of a slidable sleeve member 112. The sleeve member extends coaxially around an outer surface of a tubing section 40 comprised by the flow regulator. The sleeve member is moveable through a range of positions from a first position in which the sleeve member covers a semi-permeable area 102 of a wall of the tubing section to a second position in which the semi-permeable area 102 is fully open. Thus by changing an axial sliding position of the sleeve member 112 a level of outflow from the flow regulator can be adjusted.

Thus, the sleeve member 112 may be configured to be manually moveable across the range of positions, to permit a user to adjust a level of outflow. For example, the user may adjust the position of the sleeve in dependence upon the size of a cuff being used with the system, for example to keep maximum airflow for larger cuffs, and to reduce the airflow for smaller cuffs.

The sleeve member thus provides a secondary degree of freedom over the flow modulation as a function of pressure, by changing a maximum outflow level from the flow modulator.

It is noted that although in the above-described examples, the semi-permeable area is provided in a wall of a tubing section, the semi-permeable area may be disposed anywhere along the fluid supply path 26 between the fluid source 24 and the bladder of the cuff. This may include an area of the fluid supply path within the cuff itself.

According to a further set of embodiments, again, the flow modulator may include a tubing piece for disposal in fluid series along the fluid supply path, and wherein at least a section of the tubing piece is flexibly deformable as a function of fluid pressure inside the section of the tubing piece. Again, the at least section of the tubing piece may include one or more outflow paths (e.g. orifices or conduits or a permeable area) to permit venting of fluid out from the fluid supply path. The pressure coupling area may be formed by an interior, fluid-facing surface of the section of the tubing piece. The flow modulator may further include a seat member mounted outside of the tubing piece and facing the one or more orifices or conduits, for example a sleeve member as illustrated in Fig. 9, and wherein the tubing piece is flexibly deformable between a relaxed state in which there is a spacing between the seat member and the one or more orifices to permit outflow of fluid, and a deformed state in which the one or more orifices are closed against the seat member. Thus here, the section of the tubing piece has the adjustable physical configuration by virtue of being deformable, and an outflow from the one or more orifices is passively regulated via expansion of the tubing toward the seat member outside the tubing.

Thus, in other words, a tubing section is provided which is flexible to allow an extension in at least one direction, preferably resulting into a bigger diameter of the tube. In the region of the tubing which is flexible, at least one orifice/hole or a permeable area is provided and sleeve/cylinder provided covering the flexible part of the tube. The cylinder is provided having a slightly larger diameter than the tubing in its relaxed state so that air can release via the hole(s) in the flexible part of the tubing. As pressure in the tubing increases, the flexible part of the tubing extends and finally pushes the flexible part of the tubing towards the rigid sleeve/cylinder. If the pressure in the tube reaches a certain threshold, the holes /orifices in the flexible part of the tube are fully closed by the sleeve/cylinder.

This arrangement could be combined in some examples with the embodiments of Fig. 9, so that passive self-regulation of the venting is achieved, but at the same time a user can adjust a maximum outflow level from the vent by axially moving the coaxial seat member 112 to cover more or less of the permeable wall.

In some embodiments, the seat member comprises a sleeve member having lesser flexibility than the section of the tubing piece, e.g. a rigid or inflexible sleeve member.

Embodiments outlined above have related primarily to arrangements utilizing a variable-outflow fluid release vent, either utilizing one or more orifices, or utilizing a semi-permeable wall area. According to a further group of embodiments, variable flow modulation can be achieved using a passively self-regulating fluid resistor arrangement.

Before illustrating some particular examples of this approach, a description of a general form embodied by this group of embodiments will first be outlined.

In this group of embodiments, the flow modulator comprises a fluid resistor for imposing a resistance against a flow of the pressurized fluid through the fluid supply path, wherein a level of resistance of the fluid resistor is variable to provide the variable level of flow modulation. In general, the fluid resistor includes a flow interruption structure for impinging on fluid flowing along the fluid supply path. The flow interruption structure has the adjustable physical configuration, and wherein adjustment of the physical configuration of the flow interruption structure varies a level of fluid resistance imposed by the fluid resistor. In at least some examples therefore, the flow interruption structure includes the pressure coupling area for coupling fluid pressure to changes in a flow modulation level of the flow modulator.

Fig. 10 schematically illustrates one example. In this embodiment, a flexible structure (e.g., a membrane) 122 disposed orthogonal to the direction of air flow in a tubing section 40 is used. The flexible structure comprises a set of holes 124 which allow air to pass from one side to the other. The flexible structure 122 spans the whole diameter of the tubing section so that air can only pass the flexible structure by traveling through the holes. Thus it will be recognized that a size of the holes determines a flow rate of air past the flexible structure. The flexible structure is connected to an interior surface of the tubing section 40. The tubing section to which the flexible structure is connected is preferably also flexible, meaning, at higher pressure, the tubing 40 diameter expands, resulting in an expansion of the flexible structure also. This expansion widens the holes 124 in the flexible structure and, as a consequence, a flow rate of air through the holes can increase. At lower air pressure in the tubing 40 the flexible structure 122 contracts, resulting in a decrease in the size of the holes, thereby imposing a higher air resistance and therefore forcing a lesser rate of air flow.

Fig. 10 (left) shows the non-expanded state. Fig. 10 (right) shows the expanded state.

Thus, as a more general principle, a flow interruption structure 122 may be provided which comprises one or more fluid flow paths 124 extending from one side of the interruption structure to the other to permit fluid to pass therethrough. The previously mentioned adjustment of the physical configuration of the interruption structure 122 may include adjustment of a cross-sectional area of said one or more fluid flow paths 124.

In some examples, and as exemplified by Fig. 10, the flow interruption structure may comprise a flexibly deformable body (such as a flexible membrane or a flexible body) 122, and wherein the fluid flow paths 124 are formed through the deformable body. The fluid flow paths 124 may be configured to flexibly deform as a function of fluid pressure inside the section of the tubing piece 40.

As also exemplified by Fig. 10, the flow modulator 34 may include a tubing piece 40 for location along the fluid supply path, and wherein at least a section of the tubing piece is flexibly deformable as a function of fluid pressure inside the section of the tubing piece; and wherein the flexibly deformable body of the fluid interruption structure 122 is mechanically coupled to an interior wall of the at least section of the tubing piece, such that deformation of the tubing wall effects a correlated deformation of the interruption structure.

In the example of Fig. 10, the fluid interruption structure 122 is configured to extend across a whole of a cross-sectional area of the fluid supply path.

In this example, the pressure coupling area may include a surface of the flow interruption structure 122 and/or may include an interior surface of the tubing section 40.

Although in the example of Fig. 10, the flexibly deformable body of the fluid interruption structure takes the form of a flexible membrane, in a variant example, this could be replaced by a thicker deformable body, having a longer axial extension in the tubing 40. An example is illustrated in Fig. 11. This example may be the same as the example of Fig. 10 except for the replacement of the flexible membrane 122 with a flexibly deformable body 142 of wider axial extension. Here, the fluid flow paths formed through the deformable body take the form of flow channels 144, as opposed to openings. Accordingly, the flow paths are formed by hollow tubes or channels 144 extending through the deformable body 142 in the example of Fig. 11. Fig. 11 (left) shows the non-expanded state, while Fig. 11 (right) shows the expanded state.

Preferably the channels 144 forming the fluid paths through the deformable body 142 may have a conical cross section across a plane parallel with a longitudinal axis of the tubing section 40, as illustrated in Fig. 11. In other words, the channels 142 through the deformable body preferably have a non-uniform diameter, wherein the diameter transitions from a first smaller diameter at an inflow point to the deformable body to a second larger diameter at an outflow point from the deformable body. The transition may be a smooth linear transition, as illustrated in Fig. 11.

Another possible variation on the embodiment of Fig. 10 is to replace the single membrane 122 with a sequence of multiple membranes or other deformable bodies, arranged one after another. Each flexible structure may comprise a different number of holes and/or different sizes of holes (or both). This allows to flexibly define the flow through the tube as a function of diametric extension of the tube (which varies as a function of fluid pressure in the tube).

Another possible variation on the embodiment of Fig. 10 or Fig. 11, is to additionally provide a tubular sleeve member, e.g. cylindrical, extending co-axially around the outside of the tubing section. The sleeve may be arranged to cover the whole of the flexible part of the tubing section or just a portion. The sleeve may be axially moveable along the tubing section, to either cover or uncover the flexible portion of the tubing section in some particular examples, either manually or automatically. The sleeve may be rigid such that when it is positioned surrounding the flexible part of the tubing section, it prevents diametric expansion of the tubing, or it may have a flexibility which is less than the flexibility of the tubing section so that it partially inhibits diametric expansion of the tubing section. Thus effectively allows for controlling whether the flow regulator is active or inactive, or allows for modifying the function of flow modulation to pressure.

In some examples, the sleeve may be axially shorter than the flexible part of the tubing section so that only one length portion of the flow paths 144 would be covered by the sleeve. This would have the effect of preventing diametric expansion of a portion of the flow paths but leaving the other portion of the flow paths free to expand.

According to any of the embodiments described above, the flow regulator may be provided as an integral part of a tubing connecting the air source to the cuff, or may be provided as an attachment or add-on and adapted to be connectable in fluid series into such an air-tube. It may alternatively be incorporated at any other point along the fluid supply path, including in a part of the cuff itself or in a housing which houses the air source for example.

As already discussed, the general inventive concept admits of a wide variety of possible applications. In general, the proposed system provides a mechanically and functionally simple way of enabling auto-adjustment of the pneumatic properties of a delivered source of pressurized air, in particular applying a flow modulation which varies as a function of fluid pressure within the system (e.g. the cuff + fluid supply path system). This is achieved advantageously without the need for a dynamically controllable pump, and without the need for an electronically controlled flow regulator and thus can be implemented even by way of retrofit of existing hemodynamic monitoring systems which use an inflatable cuff.

There will now be discussed one particular use case for which the inventors have found the proposed apparatus particularly advantageous.

By way of background, an improved device developed by the inventors associated with the present application makes use of a novel cuff design, the use of which enables continuous monitoring. One problem encountered however is that the cuff design requires different sized cuffs to be used for patients with different arm sizes, most notably adults versus children.

In more detail, the improved cuff design comprises the following components. The cuff comprises an inflatable bladder, which may otherwise be referred to as the "actuator". This is supplied by an air hose or tubing. When inflated, the actuator compresses the cuff around the patient's upper arm (or any other limb).

A relatively rigid shell portion, referred to herein as the "shell", is surrounded and bound by the actuator. When the bladder is inflated, the shell portion serves to compress the patient's arm evenly and smoothly. The interior of the shell, when assembled, may be covered by a liner to prevent pinching of the patient. A sensor assembly is further included which comprises a fluid-filled pouch, referred herein as the sensor pad (SP), located so as to be between the shell portion and the limb during use of the cuff, i.e. on the interior side of the shell. This is connected by a fluid-filled tubing to a pressure transducer ("PT"). This may be disposable. It may be mountable on the exterior of the cuff. It may be connected by an electrical cable to the control module, e.g. to a patient monitor. When the actuator is inflated, the shell presses the sensor pad against the patient's inner upper arm, and the tissue pressure of the patient's arm is hydraulically conducted to the PT. The PT transduces the fluid pressure to an analog electrical signal which is conducted by means of the electrical cable to the control module, where the brachial arterial pulsation (beat-to-beat blood flow) is displayed and analyzed.

According to at least one set of examples, this cuff is a non-sterile, single-patient-use device, intended to be used on a single patient for no more than three days (72 hours). According to at least one set of examples, the cuff is intended for use on adult critical care patients in the operating room (OR) or intensive care unit (ICU) who are intubated and require fluid management.

The cuff enables non-invasive monitoring of blood pressure and a variety of other different hemodynamic parameters.

In use, the cuff is placed on the patient's upper arm and is connected to a control module, e.g. a patient monitor, by means of an electrical cable and an air hose or tubing. With regards to the electrical cable, optionally, the pressure transducer may contain an authentication circuitry (e.g. a 1-wire authentication circuitry) to identify the cuff model and other information stored therein.

According to at least one set of examples, the cuff is available in four different cuff sizes to accommodate different arm sizes. These include: small adult (SA), adult (A), large adult (LA), and large adult short (LA-S). For each of these, the volume size of the inflatable bladder is different. Some of the cuffs differ in respect of the diameter of arm that they are designed to fit to.

In order to obtain accurate measurements, a relatively slow airflow is required at a beginning of the inflation cycle. For smaller cuff sizes (e.g. Small Adult), the flow rate provided by the pump during the beginning of the inflation cycle is too high, causing the cuff to inflate too quickly, due to the smaller bladder volume compared to the larger cuffs.

To compensate for this, the inventors have recognized that there would be value in providing a flow modulator in line along the airflow path to reduce the flow rate when the system is being used with a smaller cuff size. However, the inventors have recognized that the reduced flow rate is only needed toward a beginning of an inflation cycle, to avoid the cuff being inflated too quickly. Toward an end of the inflation cycle, approximately the same pressure in the system is needed in order to keep the cuff inflated and thus little or no flow reduction is needed at this later point in the cycle. The inventors have thus recognized that there would be value in providing a flow modulator which imposes a reduction in flow rate, but wherein the level of flow reduction imposed decreases as pressure in the fluid supply path, and the overall system, increases. The inventors have provided a solution which allows for a passive self-regulating flow modulator which is capable of solving this problem, wherein the level of flow modulation decreases as a function of changing fluid pressure. The inventors have further recognized that the same principle has application beyond the particular use case described above, and that a passive self-regulating flow modulator in accordance with embodiments described above may be used advantageously in a variety of applications.

This notwithstanding, in one particular set of embodiments, the apparatus includes a first and second cuff, the second cuff having a smaller (volume) inflatable bladder than the first; and wherein the apparatus is selectively operable using either the first or second cuff connected to the fluid supply path.

In some embodiments, the or each cuff may comprise a tissue pressure sensor arranged for sensing a pressure between a surface of the body part and the cuff.

In some embodiments, the or each cuff may include a shell portion wherein the shell portion is arranged so as to be located between the bladder and the body part when the cuff is wrapped around the body part. The shell portion may be arranged to surround the body part when the cuff is wrapped around the body part.

In some embodiments, the apparatus further includes a measurement module for processing a pressure signal output by the cuff and generating one or more blood pressure measurements. This may comprise one or more processors.

In some embodiments, the apparatus further includes an inflation controller for controlling an inflation cycle of the cuff. For example the apparatus may include a control module. The control module may house the source of pressurized air (e.g. air pump), and may further house the inflation controller. The inflation controller may control the operation of the air pump in accordance with a pre-defined control program for inflating the cuff.

The one or more processors can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A flow regulator for use with a hemodynamic parameter measurement apparatus (20),
the apparatus comprising: a source (24) of pressurized fluid, a measurement cuff (22) comprising an inflatable bladder, and a fluid supply path (26) for conveying pressurized fluid from the source of pressurized fluid to the bladder of the measurement cuff; and
the flow regulator (32) being for disposal along the fluid supply path between the pressurized fluid source and the inflatable bladder of the measurement cuff,
wherein the flow regulator comprises: a flow modulator (34) for modulating a flow rate of fluid flowing along the fluid supply path, and wherein the flow modulator is configurable to provide a variable level of flow modulation across a range between a minimum level of flow modulation and a maximum level of flow modulation,
wherein at least a portion of the flow modulator has an adjustable physical configuration, wherein adjustment of the physical configuration varies the level of flow modulation;
wherein the flow modulator includes a pressure coupling area arranged to be in pressure-coupled relationship with the pressurized fluid (44) flowing along the fluid supply path, and
wherein said physical configuration of the at least portion of the flow modulator varies passively as a function of the pressure acting on the pressure coupling area.

2. The flow regulator of claim 1, wherein the physical configuration of the at least portion of the flow modulator varies as a function of the pressure acting on the pressure coupling area such that the level of flow modulation decreases as a function of pressure.

3. The flow regulator of claim 1 or 2, wherein the adjustable physical configuration is an adjustable position of at least a part of said at least portion of the flow modulator, or wherein the adjustable physical configuration is an adjustable dimension of at least a part of said at least portion of flow modulator.

4. The flow regulator of any of claims 1-3,
wherein flow modulator comprises a fluid release vent for venting fluid out from the pressurized fluid supply path, wherein an outflow level from the vent is variable to provide the variable level of flow modulation,
wherein the fluid release vent comprises a closure structure (46), the closure structure having said adjustable physical configuration, wherein adjustment of the physical configuration of the closure structure varies an outflow level from the vent;
wherein the closure structure includes said pressure coupling area.

5. The flow regulator of claim 4,
wherein the fluid release vent comprises an orifice (42) for outflow of fluid;
wherein the closure structure (46) comprises a seat member (52) for seating over the orifice to seal or release the orifice to a variable extent;
wherein the adjustable physical configuration of the closure structure comprises an adjustable positioning of the seat member relative to the orifice, wherein the seat member is variable across a range of positions for thereby varying an outflow level from the vent.

6. The flow regulator of claim 5, wherein the pressure coupling area is an area of a moveable member (54, 84, 94, 106, 107, 108), the moveable member arranged to move by a variable amount related to an applied pressure from the fluid, and wherein the movement of the moveable member is coupled directly or indirectly to the seat member (52), and
optionally wherein the closure structure comprises a biasing element (62) for biasing the seat member into an open position relative to the orifice (42) in the absence of counter-force, and wherein the movement of the moveable member responsive to applied pressure acts to counter the biasing element to move the seat member into a more closed position.

7. The flow regulator of claim 6, wherein
the moveable member comprises a flexible membrane (54); or
the moveable member comprises a linearly moveable plate (84).

8. The flow regulator of claim 6 or 7, wherein the moveable member (54) forms the seat member.

9. The flow regulator of any of claims 6-8, wherein the moveable member (54, 84) is in pressure-coupled arrangement on one side (56) with the pressurized fluid (44) flowing along the fluid supply path and is in pressure-coupled arrangement on an opposite side with a reference pressure, e.g. an ambient air pressure, whereby a pressure differential is established across said two sides of the moveable member.

10. The flow regulator of claim 6, wherein the moveable member (94) comprises a flap and wherein the flap carries a seat member (52) for seating against an orifice (42).

11. The flow regulator of any of claims 1-5,
wherein the flow modulator (34) includes a tubing piece (40) for location along the fluid supply path (26),
wherein at least a section of the tubing piece is flexibly deformable as a function of fluid pressure inside the section of the tubing piece;
wherein the at least section of the tubing piece includes one or more outflow paths (102) to permit venting of fluid out from the fluid supply path;
wherein the pressure coupling area is formed by an interior, fluid-facing surface of the section of the tubing piece;
wherein the flow modulator further includes a seat member mounted outside of the tubing piece and facing the one or more outflow paths;
wherein the tubing piece is flexibly deformable between a relaxed state in which there is a spacing between the seat member and the one or more outflow paths to permit outflow of fluid, and a deformed state in which the one or more outflow paths are closed against the seat member, and
optionally wherein the seat member comprises a sleeve member.

12. The flow regulator of any of claims 1-3,
wherein the flow modulator comprises a fluid resistor for imposing a resistance against a flow of the pressurized fluid through the fluid supply path, wherein a level of resistance of the fluid resistor is variable to provide the variable level of flow modulation;
wherein the fluid resistor includes a flow interruption structure (122, 142) for impinging on fluid flowing along the fluid supply path, the flow interruption structure having said adjustable physical configuration, wherein adjustment of the physical configuration of the flow interruption structure varies a level of fluid resistance imposed by the fluid resistor; and
optionally wherein the flow interruption structure includes said pressure coupling area.

13. The flow regulator of claim 12, wherein the flow interruption structure (122, 142) comprises one or more fluid flow paths (124, 144) extending from one side of the interruption structure to another to permit fluid to pass therethrough, and wherein the adjustment of the physical configuration of the flow interruption structure includes adjustment of a cross-sectional area of said one or more fluid flow paths.

14. The flow regulator of claim 13, wherein the flow interruption structure (122, 142) comprises a flexibly deformable body, the fluid flow paths (124, 144) being formed through the deformable body, and wherein the fluid flow paths are configured to flexibly deform as a function of fluid pressure inside the section of the tubing piece, and
optionally wherein the flexibly deformable body of the flow interruption structure is mechanically coupled to an interior wall of the at least section of the tubing piece, such that deformation of the tubing wall effects a correlated deformation of the flow interruption structure.

15. A hemodynamic parameter measurement apparatus (20), comprising:
a source (24) of pressurized fluid;
a measurement cuff (22) comprising an inflatable bladder;
a fluid supply path (26) for conveying pressurized fluid from the source of pressurized fluid to the bladder of the measurement cuff; and
a flow regulator in accordance with any preceding claim for disposal along the fluid supply path between the pressurized fluid source and the inflatable bladder of the measurement cuff, and
optionally wherein the apparatus includes a first and second cuff, the second cuff having a smaller bladder than the first, and the apparatus is selectively operable using either the first or second cuff connected to the fluid supply path.
